# FASCICULE DE BREVET EUROPEEN

(11) **EP 2 932 442 B1**
(45) Date de publication et mention de la délivrance du brevet: **01.03.2017**
(21) Numéro de dépôt: 13824601.2
(22) Date de dépôt: 11.12.2013
(51) Int. Cl.: G06M 1/08, B65D 83/22

(54) **PIÈCE MOULÉE EN MATÉRIAU SYNTHÉTIQUE**
KUNSTSTOFFFORMTEILS
MOULDED PIECE MADE OF SYNTHETIC MATERIAL

(30) Priorité: 11.12.2012 FR 1261890
(43) Date de publication de la demande: 21.10.2015
(73) Titulaire: Aptar France SAS, 27110 Le Neubourg (FR)
(72) Inventeur: PAPET, Gérard, F-76300 Sotteville Les Rouen (FR); SAUSSAYE, Anthony, F-27120 Menilles (FR)
(74) Mandataire: CAPRI
(86) Numéro de dépôt international: PCT/FR2013/053025
(87) Numéro de publication internationale: WO 2014/091150

(56) Documents cités:
- WO-A2-2005/017463
- DE-A1-102011 005 075
- FR-A1- 2 533 895

## Description

La présente invention concerne une pièce moulée en matériau synthétique, et plus particulièrement un actionneur d'un compteur ou indicateur de doses d'un dispositif de distribution de produit fluide, tel qu'un inhalateur.

Les actionneurs de compteurs sont des pièces complexes comportant généralement au moins une partie mobile. Cette partie mobile est déplacée ou déformée lors de l'actionnement pour ainsi entrainer un élément de comptage du compteur. La fiabilité des compteurs de doses étant essentielles, notamment pour des dispositifs de distribution de médicaments, ces actionneurs doivent garantir un parfait actionnement du compteur à chaque actionnement du dispositif. Le document WO2005017463 décrit un tel compteur ou indicateur de doses.

Or, comme pour toute pièce plastique moulée, il peut exister un risque de déformation de la ou des parties mobiles de l'actionneur, en particulier en sortie de moule, pendant le transport et le stockage, et enfin pendant l'assemblage. Pour limiter ces risques, les actionneurs sont traités avec un soin particulier, ce qui augmente notamment leur coût de fabrication et de conditionnement.

Les documents DE 10 2011 00575 et FR 2 533 895 décrivent des dispositifs de l'art antérieur.

La présente invention a pour but de fournir une pièce moulée en matériau synthétique, en particulier un actionneur de compteur ou indicateur de doses, qui ne reproduit pas les inconvénients susmentionnés.

En particulier, la présente invention a pour but de fournir une telle pièce moulée en matériau synthétique qui soit simple et peu coûteuse à fabriquer et à assembler, et qui puisse notamment être appliquée à tous les dispositifs de distribution de produit fluide existants sans impliquer une modification de la procédure d'assemblage.

La présente invention a donc pour objet une pièce moulée en matériau synthétique, comportant un châssis rigide et au moins une partie mobile déplaçable et/ou déformable par rapport audit châssis rigide, ladite au moins une partie mobile étant reliée lors du moulage audit châssis rigide par au moins un pont de matière sécable ledit au moins un pont de matière sécable étant cassé lors de l'assemblage de ladite pièce moulée ou lors de la première utilisation de ladite pièce moulée, ladite pièce moulée étant un actionneur d'un compteur ou indicateur de doses d'un dispositif de distribution de produit fluide, ledit actionneur comportant un châssis rigide et au moins une patte flexible pivotante par rapport audit châssis rigide, au moins un pont de matière étant formé entre ledit châssis rigide et ladite patte flexible.

Avantageusement, ledit actionneur comporte un élément de transmission déplaçable en translation par rapport audit châssis rigide et adapté à coopérer avec une partie dudit dispositif de distribution à chaque actionnement de celui-ci.

Avantageusement, ladite patte flexible comportant une première partie de patte flexible et une seconde partie de patte flexible plus rigide que la première partie de patte flexible, la première partie de patte supportant une dent d'actionnement et la seconde partie de patte supportant l'élément de transmission.

Avantageusement, ladite seconde partie de patte flexible comporte deux branches formant une structure ovoïde ayant deux sommets opposés formés d'une part par l'élément de transmission et d'autre part par une jonction à la première partie de patte flexible, ladite structure ovoïde pouvant être étirée par déplacement dudit élément de transmission et revenant élastiquement vers sa position de repos lorsque l'élément de transmission n'est plus sollicité.

Avantageusement, au moins un pont de matière est formé entre ledit châssis rigide et ladite seconde partie de patte flexible et/ou ledit élément de transmission de ladite patte flexible.

La présente invention a aussi pour objet un indicateur de doses pour dispositif de distribution de produit fluide, comportant un actionneur tel que décrit ci-dessus.

La présente invention a aussi pour objet un dispositif de distribution de produit fluide comportant un réservoir de produit et un organe de distribution, tel qu'une pompe ou une valve, monté sur ledit réservoir, comportant un tel indicateur de doses.

Avantageusement, ledit au moins un pont sécable est cassé lors de l'assemblage dudit actionneur ou lors du premier actionnement dudit dispositif de distribution.

D'autres caractéristiques et avantages de la présente invention apparaîtront plus clairement au cours de la description détaillée suivante d'un mode de réalisation particulier de celle-ci, faite en référence aux dessins joints, donnés à titre d'exemples non limitatifs, et sur lesquels,
- la figure 1 est une vue schématique en perspective d'un dispositif de distribution de produit fluide auquel la présente invention peut s'appliquer,
- la figure 2 est une vue en section transversale de coté du dispositif de la figure 1,
- la figure 3 est une vue schématique de face d'un actionneur selon un mode de réalisation avantageux de l'invention, en position de stockage, et
- la figure 4 est une vue similaire à celle de la figure 3, en position d'actionnement.

La présente invention s'applique à tout type de pièce moulée en matériau synthétique pourvue d'au moins un élément flexible, tel qu'une patte déformable élastiquement, solidaire d'une structure fixe. La description ci-après sera toutefois plus précisément faite en référence à un actionneur particulier d'un indicateur de doses pour un dispositif de distribution de produit fluide. Il est toutefois entendu que la présente invention ne se limite pas à un tel actionneur.

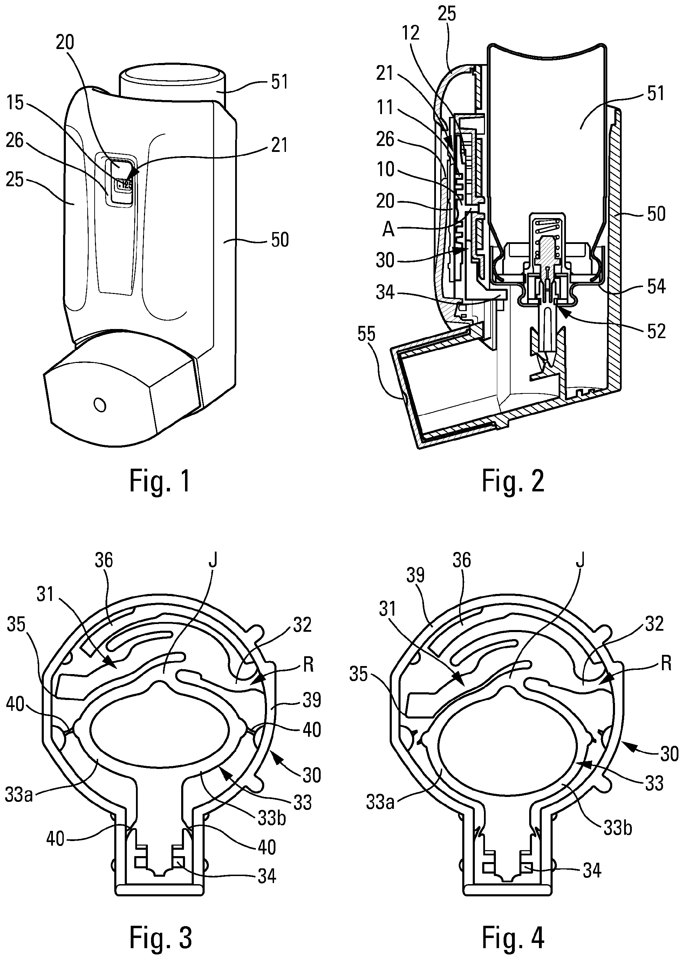

Les figures 1 et 2 représentent schématiquement un dispositif de distribution auquel la présente invention s'applique plus particulièrement. Ce dispositif d'inhalation comporte un corps 50 et un réservoir 51 sur lequel est assemblée une valve doseuse 52. L'actionnement du dispositif est obtenu par déplacement axial du réservoir 51 à l'intérieur du corps 50, ce déplacement entraînant une compression de la soupape de la valve 52 ce qui provoque l'expulsion d'une dose de produit à travers un orifice buccal 55. Bien entendu, la présente invention s'applique également à d'autres types de dispositifs de distribution, et notamment des dispositifs de pulvérisation du type nasal, ou des dispositifs comportant une pompe en lieu et place de la valve.

Le dispositif de distribution comporte un indicateur de doses qui comprend au moins un moyen de comptage rotatif 10. Dans l'exemple représenté, ce moyen de comptage est formé par un disque de comptage rotatif adapté à se déplacer en rotation autour d'un axe de rotation A sensiblement perpendiculaire audit disque. Ce disque rotatif est de préférence mince et pourvu d'un profil creux 11, qui peut avantageusement être réalisé au moyen d'une nervure ou rainure. Le disque comporte en outre avantageusement une denture 12, de préférence réalisée sur sa périphérie, ladite denture étant adaptée à coopérer avec un actionneur 30 qui est adapté à faire tourner ledit disque, et qui sera décrit plus amplement ci-après. Le disque ou roue de comptage comporte également des moyens d'indication 15, qui peuvent être des nombres et/ou des symboles, et qui sont destinés à indiquer le nombre de doses distribuées ou restant à distribuer, comme visible sur la figure 1. Bien entendu, la présente invention s'applique aussi à des compteurs ou indicateurs de doses différents.

L'indicateur représenté sur la figure 2 peut aussi comporter avantageusement un organe translatif 20, adapté à se déplacer en translation. Cet organe translatif peut comporter une projection, ou tout autre moyen équivalent, qui coopère avec ledit profil creux 11 du disque rotatif 10. Cet organe translatif 20 est de préférence réalisé sous la forme d'une plaque mince, et comporte une ouverture de visualisation 21 destinée à coopérer avec les moyens d'indication du disque rotatif.

Selon la forme du profil creux 11, une rotation de la roue de comptage 10 entraînera une translation de l'organe translatif 20. Avantageusement, la roue de comptage et l'organe translatif sont disposés dans un couvercle 25, qui est de préférence également de structure mince, et qui comporte une fenêtre de visualisation 26, coopérant avec l'ouverture de visualisation 21 de l'organe translatif pour permettre à l'utilisateur de visualiser les moyens d'indication de la route de comptage 10.

L'actionnement de l'indicateur, et en particulier la rotation de la roue de comptage rotative 10, peut avantageusement être réalisé par un actionneur 30 dudit indicateur.

Les figures 3 et 4 montrent un actionneur 30 adapté au dispositif des figures 1 et 2. Cet actionneur 30, similaire à celui décrit dans le document WO2005017463, peut avantageusement comporter un élément d'entraînement réalisé sous la forme d'une patte flexible 31, solidaire d'un châssis rigide 39. Cette patte flexible 31 est adaptée à coopérer avec ladite denture à chaque fois qu'une dose est distribuée, de préférence au moyen d'une dent 35. Avantageusement, on prévoit des moyens anti-retour 36 pour empêcher ledit disque rotatif 10 de tourner dans le sens inverse à celui qui lui est donné par la patte flexible 31 lors de l'actionnement. Ces moyens anti-retour peuvent comporter une patte flexible 36 supportant une dent anti-retour coopérant avec la denture.

L'actionneur 30 comporte également un élément de transmission 34 qui est adapté à coopérer avec le dispositif de distribution de produit fluide, à chaque actionnement de celui-ci, ledit élément de transmission 34 coopérant d'autre part avec ladite patte flexible 31 pour faire tourner ledit disque rotatif 10. En particulier, ledit élément de transmission 34 est un épaulement qui coopère avec une partie 54 du dispositif de distribution de produit fluide qui est mobile pendant l'actionnement. Dans l'exemple représenté, il s'agit de la bague de fixation 54 de la valve doseuse 52 sur le réservoir 51. Bien entendu, et plus généralement, toute partie qui se déplace lors de l'actionnement du dispositif est adaptée à coopérer avec l'épaulement 34 pour actionner l'indicateur de doses.

Avantageusement, la patte flexible 31 peut être pourvue de deux parties flexibles 32 et 33 de flexibilité différente, la première partie 32 étant plus flexible que la seconde partie 33. La seconde partie de patte 33 supporte ledit épaulement 34, et lorsque le dispositif de distribution est actionné, la bague de fixation 54 du réservoir entraîne d'abord la partie plus flexible 32 du bras 31 à se fléchir, ce qui provoque la rotation dudit disque de comptage au moyen de la dent d'actionnement 35 qui coopère avec la denture. Ainsi, pendant l'actionnement, l'épaulement 34 est déplacé en translation vers le bas (dans la position représentée sur les figures 3 et 4) par le dispositif de distribution, et la partie de bras plus flexible 32 se fléchit, faisant pivoter la patte flexible 31. Ceci entraîne la rotation de la roue de comptage sur l'équivalent d'une dent de la denture 11. La première partie de patte plus flexible 32 est alors bloquée, et une poursuite de la course d'actionnement du dispositif de distribution est possible par la flexion de la partie de patte moins flexible 33. De cette manière, on permet un actionnement de l'indicateur de doses dans la première partie de ladite course d'actionnement. Ceci élimine tout risque de non comptage d'une dose distribuée (partiellement ou totalement) en cas d'actionnement partiel du dispositif de distribution, tout en permettant une poursuite de la course d'actionnement après comptage.

Avantageusement, il est prévu des moyens d'amplification adaptés à amplifier le déplacement de l'élément de transmission 34 au tout début de la course d'actionnement, pour que le déplacement de la dent 35 soit supérieur au déplacement dudit élément de transmission 34. Dans l'exemple représenté, la seconde partie de patte 33 comporte avantageusement deux branches 33a et 33b. Ces branches sont de préférence convexes et fixées d'une part à la première partie de patte 32 au niveau d'une jonction J, et d'autre part à l'élément de transmission 34. Comme visible sur les figures 3 et 4, ces branches 33a et 33b peuvent former une structure ovoïde avec deux sommets opposés, l'un formé par ladite jonction J, l'autre formé par ledit élément de transmission 34. Un déplacement de l'élément de transmission 34 provoque donc un étirement de cette structure ovoïde, qui tire sur la première partie de patte 32. En début de course d'actionnement, l'élément de transmission 34 est déplacé vers le bas sur la figure 3. L'élément de transmission 34, et donc la seconde partie de patte 33, se déplacent en translation, sans déformation de la seconde partie de patte 33, qui est plus rigide. Seule la première partie de patte 32, plus flexible, est déplacée en rotation. La jonction J étant alignée avec l'élément de transmission implique que cette jonction J effectue la même translation que ledit élément de transmission 34 en début d'actionnement, tant que la seconde partie de patte 33 ne se déforme pas. Par conséquent, cette jonction J étant décalée par rapport à l'axe de rotation R de la première partie de patte 32, et la dent 35 étant située de l'autre côté de la jonction J par rapport à cet axe de rotation R, provoque une amplification du déplacement de la dent 35 par rapport au déplacement de la jonction J. Dans cet exemple, où la jonction est environ au centre de la première partie de patte 32, le facteur d'amplification est environ de 2. Bien entendu, en modifiant la position de la jonction J, on peut modifier le facteur d'amplification, sachant qu'il sera toujours supérieur à 1. Avantageusement, les branches 33a et 33b reviennent élastiquement vers leur position de repos après actionnement.

Bien entendu, la structure élastique à deux branches convexes pourrait être remplacée par une quelconque structure élastique monobranche ou multibranche de forme quelconque. L'essentiel pour cet actionneur particulier est que cette structure soit fixée à la première partie de patte 32 et qu'elle soit élastiquement déformable pour d'une part provoquer le déplacement rotatif de la première partie de patte en début de course d'actionnement, et d'autre part permettre la poursuite de la course d'actionnement jusqu'à son terme.

On constate donc que ce type d'actionneur doit être particulièrement précis et fiable pour éviter tout dysfonctionnement de l'indicateur. Il est donc souhaitable d'empêcher autant que possible les risques de déformation des parties flexibles dudit actionneur avant son utilisation, et en particulier pendant sa fabrication, son stockage, son transport et son assemblage.

La présente invention prévoit donc de fixer les parties mobiles de l'actionneur à la bague rigide 39 de celui-ci par au moins un pont de matière sécable 40. Ainsi, au moins un pont de matière 40 est formé entre ledit châssis rigide 39 et ladite patte flexible 31. Avantageusement, ce pont de matière 40 est formé au niveau de ladite seconde partie de patte flexible 33 et/ou au niveau dudit élément de transmission 34. Avantageusement plusieurs ponts de matière peuvent être prévus, par exemple quatre comme représenté sur la figure 3.

Ainsi, lors du moulage, l'élément de transmission 34 et les branches 33a et 33b peuvent être fixées au châssis rigide 39, ce qui bloque toute déformation des ces parties flexibles et/ou déformables par rapport audit châssis, que ce soit en translation, en pivotement ou en gauchissement hors du plan de l'actionneur. Bien entendu, le ou les ponts de matière peuvent être prévu(s) à tout endroit approprié de l'actionneur.

Pour utiliser l'actionneur, la solution la plus simple est de casser ces ponts de matière lors du premier actionnement du dispositif. La force exercée par l'utilisateur pour déplacer le réservoir 51 dans le corps 50 et ainsi actionner la valve 52 sera dans ce cas suffisante pour casser le ou les ponts de matière 40. Dans cette hypothèse, la présence des ponts de matière ne modifie pas la procédure d'assemblage de l'actionneur, ce qui est particulièrement avantageux.

En variante, on peut prévoir de couper les ponts de matière 40 lors de l'assemblage de l'indicateur de dose. En effet, une fois assemblé dans l'indicateur, les risques de déformation de l'actionneur 30 sont beaucoup plus faibles que lors des phases précédentes de fabrication, de stockage ou de transport. Tout moyen de découpe approprié est envisageable, tel que par exemple une découpe au laser. Cette mise en oeuvre rend l'assemblage de l'actionneur un peu plus complexe, mais ceci peut se justifier dans certains cas.

La présente invention permet donc d'obtenir notamment les avantages suivants :
- supprimer les déformations des éléments flexibles de l'actionneur lors de son moulage, et en particulier après l'éjection hors du moule,
- supprimer les déformations potentielles dues aux manipulations, au transfert et à l'emballage de l'actionneur après moulage,
- réduire les coûts de l'emballage et du stockage et simplifier ces opérations, et
- améliorer la robustesse du processus d'assemblage de l'indicateur, en particulier de l'actionneur.

De manière plus générale, la présente invention concerne toute pièce moulée comportant au moins une partie déplaçable et/ou déformable par rapport à une partie rigide, et propose de limiter voire empêcher toute déformation de ladite partie flexible avant son assemblage et/ou avant sa première utilisation.

La présente invention a été décrite en référence à un mode de réalisation particulier de celle-ci, représenté sur les dessins, mais elle n'est aucunement limitée à cette forme de réalisation particulière. Au contraire, un homme du métier peut y apporter toutes modifications sans sortir du cadre de la présente invention tel que défini dans les revendications annexées.

## Revendications

1. Pièce moulée (30) en matériau synthétique, comportant un châssis rigide (39) et au moins une partie mobile (31 ; 32 ; 33 ; 34) déplaçable et/ou déformable par rapport audit châssis rigide (39), ladite au moins une partie mobile étant reliée lors du moulage audit châssis rigide par au moins un pont de matière sécable (40) ledit au moins un pont de matière sécable étant apte à être cassé lors de l'assemblage de ladite
pièce moulée ou lors de la première utilisation de ladite pièce moulée, **caractérisé en ce que** ladite pièce moulée est un actionneur d'un compteur ou indicateur de doses d'un dispositif de distribution de produit fluide, ledit actionneur (30) comportant un châssis rigide (39) et au moins une patte flexible (31) pivotante par rapport audit châssis rigide (39), au moins un pont de matière (40) étant formé entre ledit châssis rigide (39) et ladite patte flexible (31).

2. Pièce moulée selon la revendication 1, dans laquelle ledit actionneur (30) comporte un élément de transmission (34) déplaçable en translation par rapport audit châssis rigide (39) et adapté à coopérer avec une partie (54) dudit dispositif de distribution à chaque actionnement de celui-ci.

3. Pièce moulée selon la revendication 1 ou 2, dans laquelle ladite patte flexible (31) comportant une première partie de patte flexible (32) et une seconde partie de patte flexible (33) plus rigide que la première partie de patte flexible (32), la première partie de patte (32) supportant une dent d'actionnement (35) et la seconde partie de patte (33) supportant l'élément de transmission (34).

4. Pièce moulée selon la revendication 3, dans laquelle ladite seconde partie de patte flexible (33) comporte deux branches (33a, 33b) formant une structure ovoïde ayant deux sommets opposés formés d'une part par l'élément de transmission (34) et d'autre part par une jonction (J) à la première partie de patte flexible (32), ladite structure ovoïde pouvant être étirée par déplacement dudit élément de transmission (34) et revenant élastiquement vers sa position de repos lorsque l'élément de transmission n'est plus sollicité.

5. Pièce moulée selon l'une quelconque des revendications précédentes, dans laquelle au moins un pont de matière (40) est formé entre ledit châssis rigide (39) et ladite seconde partie de patte flexible (33) et/ou ledit élément de transmission (34) de ladite patte flexible (31).

6. Indicateur de doses pour dispositif de distribution de produit fluide, **caractérisé en ce qu'**il comporte un actionneur (30) selon l'une quelconque des revendications précédentes.

7. Dispositif de distribution de produit fluide comportant un réservoir de produit (51) et un organe de distribution (52), tel qu'une pompe ou une valve, monté sur ledit réservoir (51), **caractérisé en ce qu'**il comporte un indicateur de doses selon la revendication 6.

8. Dispositif selon la revendication 7 dans lequel ledit au moins un pont sécable (40) est cassé lors de l'assemblage dudit actionneur (30) ou lors du premier actionnement dudit dispositif de distribution.

## Patentansprüche

1. Formteil (30) aus Kunststoff, das einen starren Grundkörper (39) und wenigstens einen beweglichen Teil (31; 32; 33; 34) umfasst, der bezüglich des starren Grundkörpers (39) verschiebbar und/oder verformbar ist, wobei dieser wenigstens eine bewegliche Teil während des Formens mit dem starren Grundkörper durch wenigstens eine Brücke (40) aus zerbrechbarem Material verbunden worden ist, und wobei diese wenigstens eine Brücke aus zerbrechbarem Material beim Zusammenbau des Formteils oder beim ersten Gebrauch des Formteils zerbrochen werden kann,
**dadurch gekennzeichnet, dass** das Formteil eine Betätigungsvorrichtung für einen Zähler oder für eine Anzeigevorrichtung für Dosen einer Abgabevorrichtung für ein fluidförmiges Produkt ist, wobei diese Betätigungsvorrichtung (30) einen starren Grundkörper (39) und wenigstens eine flexible Klaue (31) umfasst, die bezüglich des starren Grundkörpers (39) verschwenkbar ist, wobei wenigstens eine Materialbrücke (40) zwischen dem starren Grundkörper (39) und der flexiblen Klaue (31) ausgebildet ist.

2. Formteil nach Anspruch 1, bei welchem die Betätigungsvorrichtung (30) ein Übertragungselement (34) umfasst, das bezüglich des starren Grundkörpers (39) translatorisch verschiebbar und geeignet ist, bei jeder Betätigung der Abgabevorrichtung mit einem Teil (54) dieser Abgabevorrichtung zusammenzuwirken.

3. Formteil nach Anspruch 1 oder 2, bei welchem die flexible Klaue (31) einen ersten flexiblen Klauenteil (32) und einen zweiten flexiblen Klauenteil (33) umfasst, der starrer als der erste flexible Klauenteil (32) ist, wobei der erste Klauenteil (32) einen Betätigungsvorsprung (35) und der zweite Klauenteil ein Übertragungselement (34) trägt.

4. Formteil nach Anspruch 3, bei welchem der zweite flexible Klauenteil (33) zwei Arme (33a, 33b) umfasst, die eine ovale Struktur bilden, welche zwei einander gegenüberliegende Scheitel aufweist, die einerseits von dem Übertragungselement (34) und andererseits von einer Verbindung (J) mit dem ersten flexiblen Klauenteil (32) gebildet werden, wobei die ovale Struktur durch eine Verschiebung des Übertragungselementes (34) gestreckt werden kann und in elastischer Weise in ihre Ruhelage zurückkehrt, wenn das Übertragungselement nicht mehr vorgespannt ist.

5. Formstück nach einem der vorhergehenden Ansprüche, bei dem wenigstens eine Materialbrücke (40) zwischen dem starren Grundkörper (39) und dem zweiten Teil des flexiblen Klauenteils (33) und/oder dem Übertragungselement (34) der flexiblen Klaue (31) ausgebildet ist.

6. Dosis-Anzeigevorrichtung für eine Abgabevorrichtung für ein fluidförmiges Produkt, **dadurch gekennzeichnet, dass** sie eine Betätigungsvorrichtung (30) nach einem der vorhergehenden Ansprüche umfasst.

7. Abgabevorrichtung für ein fluidförmiges Produkt, die einen Produktbehälter (51) und ein Abgabeorgan (52), wie zum Beispiel eine Pumpe oder ein Ventil umfasst, die bzw. das auf dem Behälter (51) montiert ist, **dadurch gekennzeichnet, dass** sie eine Dosis-Anzeigevorrichtung nach Anspruch 6 umfasst.

8. Vorrichtung nach Anspruch 7, bei welcher wenigstens eine zerbrechbare Brücke (40) beim Zusammenbau der Betätigungsvorrichtung (30) oder bei der ersten Betätigung der Abgabevorrichtung zerbrochen wird.

## Claims

1. A molded part (30) that is made of synthetic material and that comprises a rigid frame (39) and at least one movable portion (31; 32; 33; 34) that is movable and/or deformable relative to said rigid frame (39), said at least one movable portion being connected, during molding, to said rigid frame via at least one breakable bridge of material (40), said at least one breakable bridge of material being adapted to be broken while said molded part is being assembled, or while said molded part is being used for the first time, said molded part being **characterized in that** it is an actuator for actuating a dose counter or indicator of a fluid dispenser device, said actuator (30) comprising a rigid frame (39) and at least one flexible tab (31) that pivots relative to said rigid frame (39), at least one bridge of material (40) being formed between said rigid frame (39) and said flexible tab (31).

2. A molded part according to claim 1, wherein said actuator (30) includes a transmission element (34) that is movable in translation relative to said rigid frame (39), and that is adapted to co-operate with a portion (54) of said dispenser device each time said device is actuated.

3. A molded part according to claim 1 or claim 2, wherein said flexible tab (31) includes a first flexible tab portion (32) and a second flexible tab portion (33) that is more rigid than the first flexible tab portion (32), the first tab portion (32) supporting an actuator tooth (35) and the second tab portion (33) supporting the transmission element (34).

4. A molded part according to claim 3, wherein said second flexible tab portion (33) comprises two branches (33a, 33b) that form an oval structure having two opposite ends that are formed firstly by the transmission element (34), and secondly by a junction (J) with the first flexible tab portion (32), said oval structure being capable of stretching by moving said transmission element (34), and of returning resiliently to its rest position when the transmission element is no longer stressed.

5. A molded part according to any preceding claim, wherein at least one bridge of material (40) is formed between said rigid frame (39) and said second flexible tab portion (33) and/or said transmission element (34) of said flexible tab (31).

6. A dose indicator for a fluid dispenser device, said dose indicator being **characterized in that** it includes an actuator (30) according to any preceding claim.

7. A fluid dispenser device comprising a fluid reservoir (51) and a dispenser member (52), such as a pump or a valve, mounted on said reservoir (51), said device being **characterized in that** it includes a dose indicator according to claim 6.

8. A device according to claim 7, wherein said at least one breakable bridge (40) is broken while said actuator (30) is being assembled, or while said dispenser device is being actuated for the first time.
